# EUROPÄISCHE PATENTANMELDUNG

(11) **EP 1 397 983 A1**
(43) Veröffentlichungstag der Anmeldung: **17.03.2004**
(21) Anmeldenummer: 02405802.6
(22) Anmeldetag: 13.09.2002
(51) Int. Cl.: A47C 21/02, A61G 7/05

(54) **Fussschutz für den Einsatz in Betten**

(71) Anmelder: Grital Group (Anstalt), 9490 Vaduz (LI)
(72) Erfinder: Tini, Paolo Giuseppe, 7000 Chur (CH)
(74) Vertreter: Wagner, Wolfgang Heribert

(57) **Zusammenfassung**

Ein sich an dessen Fussende über die ganze Breite eines Bettes (4) erstreckender Fussschutz (1), der die Füsse diesbezüglich empfindlicher Patienten gegen Belastung durch die Bettdecke schützt, ist als etwa quaderförmiger Kasten ausgebildet, der zwischen einem Fussteil (3) des Bettes (4) und dem Fussende einer Matratze (9) auf dem Bettgestell (7) abgestützt ist. Ein gegen das Kopfende des Bettes (4) vorstehender waagrechter Stützflansch (10) liegt dabei auf der Matratzenoberfläche auf. Die Füsse (15; 15') des Patienten können sowohl bei Rückenlage (15) als auch bei Bauchlage (15') ihre natürliche Lage einnehmen und sind nicht belastet. Der Fussschutz (1) besteht aus Kunststoff und ist mit wärmedämmendem Material wie Schaumstoff, Velours oder Filz ausgekleidet. Die Farbe der den Füssen zugewandten Innenseite kann nach chromotherapeutischen Gesichtspunkten gewählt und z.B. weiss, schwarz, grün, gelb oder rot sein. Zur Störfeldbehebung und Beeinflussung der Akupunkturmeridiane der traditionellen chinesischen Medizin können Magnete eingebaut sein.

## Beschreibung

### Technisches Gebiet

Die Erfindung betrifft einen Fussschutz für den Einsatz in Betten. Er kann vor allem in Spitälern zum Schutz der Füsse eines Patienten vor dem Gewicht der Bettdecke am Ende der Matratze angeordnet werden.

### Stand der Technik

Bei vielen Symptomen wäre es sehr nützlich, wenn die Füsse eines Patienten, vor allem, wenn er völlig ans Bett gefesselt ist, vor der ständigen Belastung durch die Bettdecke geschützt werden könnten. Dazu gibt es aber keine brauchbaren Vorrichtungen. Wohl sind Schutzvorrichtungen für die Füsse bekannt, die Schutz vor Wundliegen und dgl. bieten sollen, doch sind sie verbandsartig ausgebildet (s.
FR-A-2 579 099) und immobilisieren daher den Patienten weitgehend. Gegen den Druck der Bettdecke bieten sie trotzdem keinen ausreichenden Schutz, wenn sie wie die in der erwähnten Schrift beschriebene Schutzvorrichtung die Zehen freilassen.

### Darstellung der Erfindung

Der Erfindung liegt die Aufgabe zu Grunde, einen Fussschutz anzugeben, welcher einen ausreichenden Schutz der Füsse gegen die Last der Bettdecke bietet, dabei aber die Bewegungsfreiheit des Patienten nicht beeinträchtigt.

Der erfindungsgemässe Fussschutz ist vor allem dort angezeigt, wo eine besondere Empfindlichkeit gegen auf die Füsse, insbesondere auf die Zehen wirkenden Druck vorliegt. Dies ist bei vielen pathologischen Erscheinungen der Fall wie bei degenerativen Erkrankungen des Bewegungsapparats, z. B. Fussarthrosen und chronischer Polyarthritits der Füsse, Grosszehenabweichungen wie Hallux, Zehenfehlstellungen wie Hammerzehen, Zehendruckstellen wie Hühneraugen oder eingewachsene Zehen, Fussschweiss bei zu heissen Füssen, bei Fusspilz und chronischen Ekzemen, arteriellen und venösen Durchblutungsstörungen der Beine oder Füsse wie zu kalte Füsse oder Raucherbein, nächtlicher Unruhe in den Beinen (restless legs), offenen Beinen, Krampfadern, Wadenkrämpfen, als Folge von Stoffwechselstörungen wie Zuckerkrankheit, Gicht oder M. Sudeck, bei Warzen, Krusten, Hautrissen an den Fersen, aber auch bei Fussverstauchungen, Fussfehlstellungen wie Senk-Hohl-, Knick-, Spreiz-, Flachfuss, Achillessehnenproblemen, Knie- und Hüftarthrosen, bei akuten und chronischen Schmerzsyndromen des Rückens wie Ischias oder Hexenschuss und bei Lähmungen wie Halbseitenlähmung nach Hirnschlag. Daneben ist auch ein Einsatz zur Entlastung der Füsse bei Beinbrüchen möglich.

Durch Störfeldbehebung im Bereich der Füsse mittels im Fussschutz eingebauter Magnete kann auch bei Schlafstörungen, Kopfschmerzen sowie Nacken- und Schulterverspannungen eine günstige Wirkung erzielt, insbesondere die Durchblutung verbessert werden. Auch im Rahmen einer Reprogrammation des Gesamttonus des Bewegungsapparats nach Dr. Bricot oder Beeinflussung der Akupunkturmeridiane der Beine (Niere, Blase, Gallenblase, Leber, Milz-Bauchspeicheldrüse, Magen) kann er mit Gewinn eingesetzt werden. Durch geeignete Wahl der Farbe nach chromotherapeutischen Gesichtspunkten können zusätzliche Wirkungen erzielt werden.

### Kurze Beschreibung der Zeichnungen

Im folgenden wird die Erfindung anhand von Figuren, welche lediglich Ausführungsbeispiele darstellen, näher erläutert. Es zeigen
- Fig. 1: eine perspektivische Ansicht eines erfindungsgemässen Fussschutzes gemäss einer ersten Ausführungsform,
- Fig. 2a: eine Ansicht des Fussschutzes nach Fig. 1 vom Kopfende des Bettes her,
- Fig. 2b: einen Schnitt längs B-B in Fig. 2a,
- Fig. 2c: eine Draufsicht auf den Fussschutz nach Fig. 1,
- Fig. 3: eine perspektivische Ansicht eines erfindungsgemässen Fussschutzes gemäss einer zweiten Ausführungsform,
- Fig. 4a: eine Ansicht des Fussschutzes nach Fig. 3 vom Kopfende des Bettes her,
- Fig. 4b: eine Seitenansicht des Fussschutzes nach Fig. 3 und
- Fig. 4c: eine Draufsicht auf den Fussschutz nach Fig. 3.

### Wege zur Ausführung der Erfindung

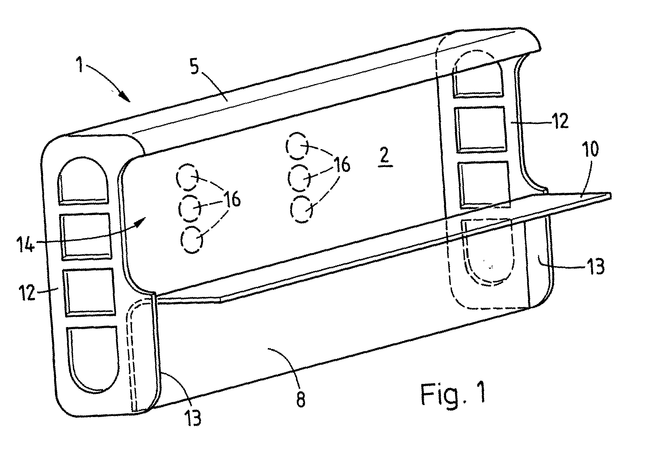

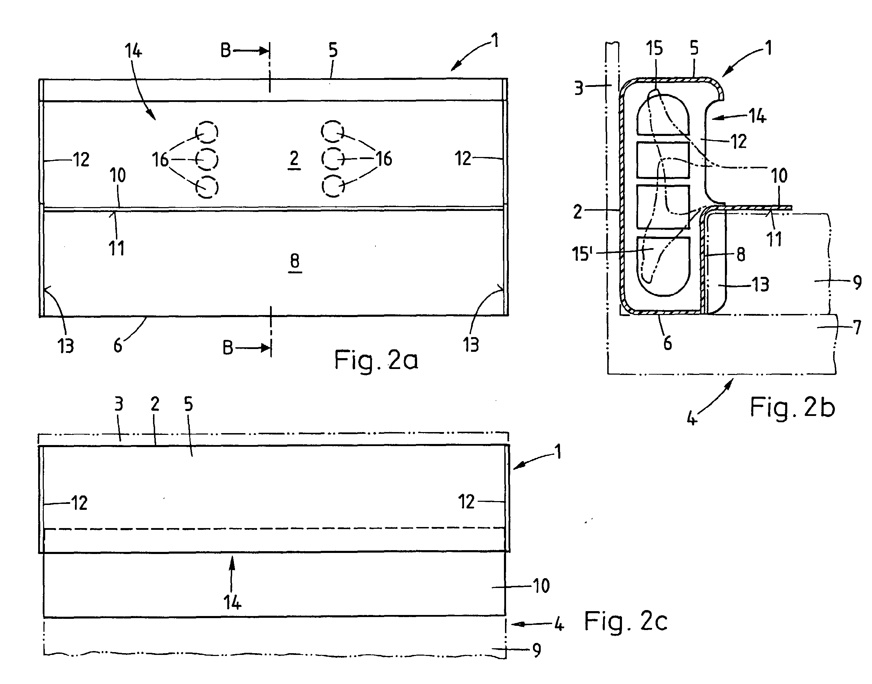

Der erfindungsgemässe Fussschutz 1 ist gemäss einer ersten Ausführungsform (Fig. 1, 2a,b,c) schalenartig aufgebaut mit einer geraden senkrechten Rückwand 2, die am Fussteil 3 des Bettes 4 anliegt und an deren oberen Rand eine Auflageplatte 5 anschliesst, die etwa waagrecht gegen das Kopfende des Bettes 4 vorragt und deren vorderer Randbereich etwas nach unten gebogen ist. An den unteren Rand der Rückwand 2 schliesst ein gegen das Kopfende des Bettes 4 gerichteter waagrechter Boden 6 an, der auf dem Bettgestell 7 aufliegt. An seinen Vorderrand schliesst eine senkrecht nach oben ragende Frontwand 8, deren Aussenseite am Fussende der Matratze 9 anliegt, an. An deren oberen Rand wiederum schliesst ein Stützflansch 10 an, welcher gegen das Kopfende des Bettes 4 vorsteht und dessen Unterseite eine Abstützfläche 11 bildet, mit der der Fussschutz 1 auf der Oberfläche der Matratze 9 aufliegt. Seitlich ist der Fussschutz 1, dessen Breite der des Bettes 4 entspricht, durch senkrechte durchbrochene Seitenplatten 12 abgeschlossen, welche beidseits der Frontplatte 8 etwas gegen das Kopfende des Bettes 4 überstehen, so dass ihre Innenseiten gegeneinander weisende Wangen 13 bilden, die seitlich an der Matratze 9 anliegen.

Der Fussschutz 1 bildet so einen etwa quaderförmigen Kasten mit über die Breite gleichbleibendem Querschnitt, der einen von der Auflageplatte 5 überdachten, gegen das Kopfende des Bettes 4 offenen Hohlraum zur Aufnahme der Füsse teilweise umschliesst. Die Oeffnung 14 erstreckt sich über seine ganze Breite, ihre Höhe ist etwas grösser ist als die Länge des Fusses des Patienten. Da er mit der Rückwand 2 am Fussteil 3, mit dem Boden 6 am Bettgestell 7, mit der Frontwand 8 am Fussende der Matratze 9 und schliesslich mit dem Stützflansch 10 an deren Oberfläche abgestützt ist, ist der Fussschutz 1 zuverlässig festgelegt, zumal die Wangen 13 eine seitliche Verschiebung verhindern. Da sich der Hohlraum von der Ebene der Oberfläche der Matratze 9 aus sowohl nach oben als auch um mindestens etwa die Länge eines Vorfusses nach unten erstreckt, bietet er den Füssen 15; 15' (Fig. 2b) des Patienten sowohl bei Rückenlage (15) als auch bei Bauchlage (15') Freiraum. In beiden Fällen können die Füsse ihre natürliche Stellung einnehmen und sind keinem auf die Zehen wirkenden Druck ausgesetzt.

Der Fussschutz 1 besteht vorzugsweise aus Kunststoff mit oder ohne Faserverstärkung. Er kann mit austauschbaren Polsterungen versehen sein, vor allem an der Oberseite des Stützflansches 10, wo die Unterschenkel des Patienten aufliegen. Die Seitenplatten 12 können mit Papier oder Gaze überzogen sein, so dass eine zu starke Auskühlung durch die Oeffnungen vermieden wird. Die Innenseite, d.h. die Unterseite der Auflageplatte 5, die Vorderseite der Rückwand 2 und auch die Oberseite des Bodens 6 und die Rückseite der Frontwand 8 sind vorzugsweise mit einem wärmedämmenden Material wie Schaumstoff, Velours oder Filz bedeckt, so dass bei Berührung kein Kältegefühl entsteht.

Zur Störfeldbehebung und Steigerung der Durchblutung durch Einwirkung auf die Fusssohlenreflexzonen und Akupunkturmeridiane der traditionellen chinesischen Medizin (TMC) können am Fussschutz auch Magnete angebracht, z.B. versenkt an der Innenseite der Rückwand 2 eingebaut sein. Im Beispiel sind zwei senkrechte Reihen von scheibenförmigen Magneten 16 von bis zu 5 cm Durchmesser so angeordnet, dass sie insbesondere auf die Nierenmeridiane an den Zehenballen beider Füsse wirken. Der Fussschutz kann auch in verschiedenen chromotherapeutisch wirksamen Farben hergestellt oder ausgekleidet sein, insbesondere schwarz, weiss, grün, gelb oder rot.

Länge und Höhe können so gewählt werden, dass sie für normale Fussgrössen auf jeden Fall ausreichen oder auch an die zu erwartenden Grössen angepasst oder auch anpassbar sein. Die Höhe - d. h. der senkrechte Abstand zwischen dem Stützflansch 10 und der Auflageplatte 5 - kann also z. B. 35 cm bis 40 cm betragen. Die Breite richtet sich vorzugsweise nach der Bettbreite oder genauer der Matratzenbreite von z. B. 95 cm, 110 cm oder mehr, obwohl auch eine geringere Breite wie z. B. 55 cm unter Umständen ausreicht, wenn die Vorsprünge der Seitenwände mit den Wangen 13 weggelassen sind.

Es sind aber auch weitergehende Abwandlungen möglich. So kann die Auflageplatte schalenartig gerundet oder durchbrochen sein. Es ist auch möglich, die Bodenplatte auf der Höhe der Matratzenoberfläche anzuordnen. In diesem Fall liegt der Fussschutz auf der Matratze auf und es braucht kein Zwischenraum zwischen dem Fussende derselben und dem Fussteil des Bettes frei zu sein. Freilich erfüllt der Fussschutz dann seinen Zweck nur bei Rückenlage des Patienten.

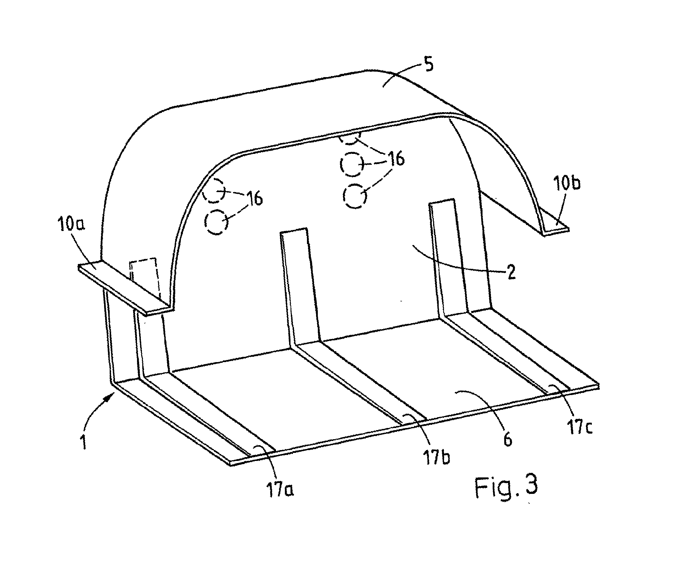

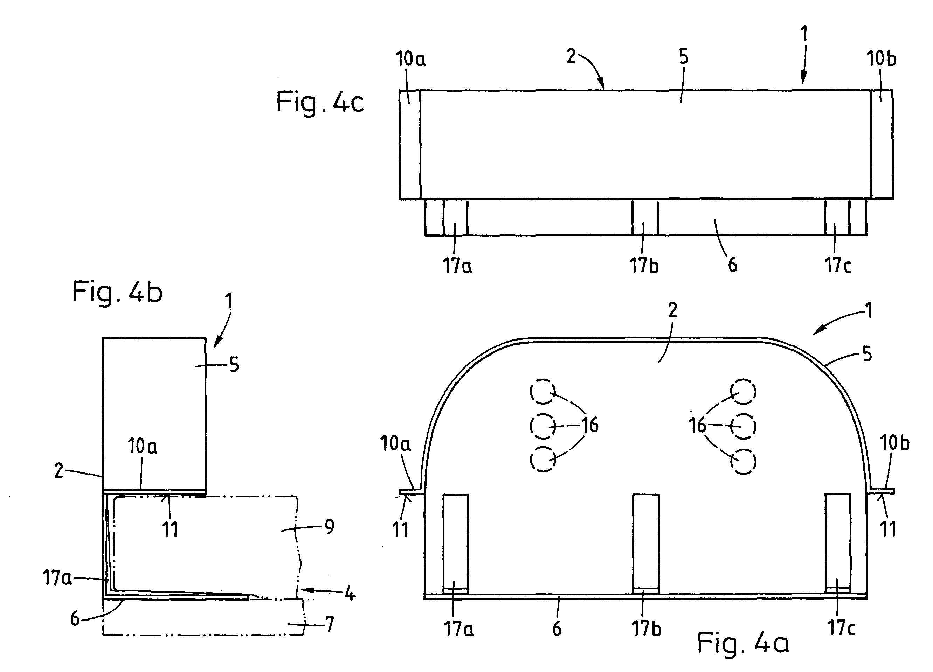

Gemäss einer zweiten Ausführungsform des Fussschutzes 1 (Fig. 3, 4a,b,c, die entsprechenden Teile sind mit den gleichen Bezugszeichen bezeichnet wie bei der ersten Ausführungsform) schliesst an den oberen Rand einer Rückwand 2, die am Fussende der Matratze 9 anliegt, eine Auflageplatte 5 an, welche ausgehend von einem ebenen Mittelteil beidseits rund nach unten gezogen ist, wo sie seitlich nach aussen abstehende Stützflansche 10a,b trägt, deren Unterseiten eine auf der Oberfläche der Matratze 9 aufliegende Abstützfläche 11 bilden. Ein vom unteren Ende der Rückwand 2 gegen das Kopfende des Bettes 4 vorstehender Boden 6 ist als ebene Platte ausgebildet, welche unter die Matratze 9 greift und auf dem Bettgestell 7 aufliegt. Drei als Ausbuchtungen ausgebildete parallele Versteifungswülste 17a,b,c erstrecken sich jeweils über einen Teil der Rückwand 2 und den Boden 6.

Es können die gleichen Materialien wie im Zusammenhang mit der ersten Ausführungsform beschrieben verwendet werden. Ausserdem können wie dort Magnete 16 eingebaut und kann die Farbe nach chromotherapeutischen Gesichtspunkten festgelegt sein. Auch die Abmessungen können mutatis mutandis entsprechend gewählt werden. Die Breite braucht jedoch nicht an die Bettbreite angepasst zu sein und kann z.B. ca. 80 cm betragen. Die zweite Ausführungsform ist einfacher als die erste. Sie schützt die Füsse lediglich bei Rückenlage des Patienten vor der Belastung durch die Bettdecke.

### Bezugszeichenliste

- 1: Fussschutz
- 2: Rückwand
- 3: Fussteil
- 4: Bett
- 5: Auflageplatte
- 6: Boden
- 7: Bettgestell
- 8: Frontwand
- 9: Matratze
- 10, 10a,b: Stützflansch
- 11: Abstützfläche
- 12: Seitenplatten
- 13: Wangen
- 14: Oeffnung
- 15, 15': Fuss
- 16: Magnet
- 17a,b,c: Verstärkungswinkel

## Patentansprüche

1. Fussschutz (1) zur Verwendung am Fussende eines Bettes (4) mit einer Matratze (9), **dadurch gekennzeichnet, dass** er eine Auflage aufweist, welche so am Bett (4) abstützbar ist, dass sie am Fussende desselben derart oberhalb der Ebene der Oberfläche der Matratze (9) gehalten ist, dass zwischen der Auflage und der besagten Ebene ein Zwischenraum freibleibt, dessen Höhe etwas grösser als die Fusslänge ist.

2. Fussschutz (1) nach Anspruch 1, **dadurch gekennzeichnet, dass** die Auflage als etwa waagrechte Auflageplatte (5) oder Auflageschale ausgebildet ist und zur Abstützung eine mit derselben verbundene waagrechte Abstützfläche (11) zur Auflage auf der Matratzenoberfläche vorgesehen ist.

3. Fussschutz (1) nach Anspruch 1 oder 2, **dadurch gekennzeichnet, dass** die Auflage an eine senkrechte Rückwand (2) anschliesst, von der sie gegen das Kopfende des Bettes (4) vorspringt.

4. Fussschutz (1) nach Anspruch 3, **dadurch gekennzeichnet, dass** unterhalb der Auflage an die Rückwand (2) ein gegen das Kopfende des Bettes (4) gerichteter waagrechter Boden (6) zur Abstützung am Bettgestell (7) zwischen einem Fussteil (3) des Bettes (4) und dem Fussende der Matratze (9) und weiter eine senkrecht nach oben vom Boden (6) abragende Frontwand (8) zur Abstützung am Fussende der Matratze (9) anschliesst.

5. Fussschutz (1) nach den Ansprüchen 2 und 4, **dadurch gekennzeichnet, dass** am oberen Ende der Frontwand (8) ein gegen das Kopfende des Bettes (4) gerichteter waagrechter Stützflansch (10) anschliesst, dessen Unterseite die Abstützfläche (11) bildet.

6. Fussschutz (1) nach Anspruch 4 oder 5, **dadurch gekennzeichnet, dass** er beidseits senkrechte Seitenwände (12) umfasst, welche an die Rückwand (2), Frontwand (8) und Auflageplatte (5) oder Auflageschale anschliessen.

7. Fussschutz (1) nach Anspruch 6, **dadurch gekennzeichnet, dass** die Seitenwände (12) durchbrochen sind.

8. Fussschutz (1) nach Anspruch 3, **dadurch gekennzeichnet, dass** unterhalb der Auflage an die Rückwand (2) ein gegen das Kopfende des Bettes (4) gerichteter waagrechter Boden (6) zur Abstützung zwischen dem Bettgestell (7) und der Matratze (9) anschliesst.

9. Fusschutz (1) nach Anspruch 8, **dadurch gekennzeichnet, dass** die Auflage beidseits nach unten gezogen ist und an ihren Rändern Stützflansche (10a, 10b) trägt, deren Unterseiten die Auflagefläche (11) bilden.

10. Fussschutz (1) nach einem der Ansprüche 1 bis 9, **dadurch gekennzeichnet, dass** er beidseits gegen das Kopfende vorspringende gegeneinanderweisende Wangen (13) zur Abstützung an den Seiten der Matratze (9) aufweist.

11. Fussschutz (1) nach einem der Ansprüche 1 bis 10, **dadurch gekennzeichnet, dass** er im wesentlichen aus Kunststoff besteht.

12. Fusschutz nach einem der Ansprüche 1 bis 11, **dadurch gekennzeichnet, dass** die den Füssen zugewandte Innenseite mindestens teilweise mit einem wärmedämmenden Material wie Schaumstoff, Velours oder Filz ausgekleidet ist.

13. Fusschutz nach einem der Ansprüche 1 bis 12, **dadurch gekennzeichnet, dass** er mindestens einen Magneten (16) aufweist.

14. Fusschutz nach Anspruch 13, **dadurch gekennzeichnet, dass** er zwei mit Abstand nebeneinander angeordnete Magnete oder im wesentlichen senkrechte Reihen von Magneten (16) aufweist.

15. Fusschutz nach einem der Ansprüche 1 bis 14, **dadurch gekennzeichnet, dass** die den Füssen zugewandte Innenseite überwiegend eine der folgenden Farben aufweist: weiss, schwarz, grün, gelb, rot.
